(19) Europäisches Patentamt
European Patent Office
Office européen des brevets

(11) EP 1 900 378 A1

(12) EUROPEAN PATENT APPLICATION

(43) Date of publication:
**19.03.2008 Bulletin 2008/12**

(21) Application number: **06119884.2**

(22) Date of filing: **31.08.2006**

(51) Int Cl.:
***A61K 45/06*** *(2006.01)* ***A61K 31/69*** *(2006.01)*
***A61K 31/137*** *(2006.01)* ***A61P 31/10*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Novartis AG**
**4056 Basel (CH)**

(72) Inventor: **Mayer, Friederich Karl**
**4104, Oberwil (CH)**

(74) Representative: **Vallet, Lucien et al**
**Novartis AG**
**Geistiges Eigentum Konzern**
**Patent- und Markenabteilung CH**
**Postfach**
**CH-4002 Basel (CH)**

# (54) Pharmaceutical compositions for the treatment of fungal infections

(57) Synergistic combinations of a squalene epoxidase inhibitor and a leucyl-tRNA synthetase inhibitor are provided, which are useful in particular in the treatment of diseases involving fungal or suspected fungal infection, for immunomodulation or immunosuppression in conditions in which fungal or suspected fungal colonisation of e.g. the skin or nail plays a role, such as atopic dermatitis, acne vulgaris, seborrhoeic dermatitis, rosacea or onychomycosis, and in situations of fungal resistance.

EP 1 900 378 A1

## Description

The invention relates to pharmaceutical compositions, for use in particular against fungal infections or inflammatory skin diseases.

It concerns a pharmaceutical composition comprising a squalene epoxidase inhibitor in combination or association with a leucyl-tRNA synthetase inhibitor.

While an antifungal activity is known for various squalene epoxidase inhibitors such as terbinafine, leucyl-tRNA synthetase inhibitors only recently have been found to constitute a novel class of antifungals with broad-spectrum activity against dermatophytes, yeasts and molds (see e.g. W. Mao et al., "AN2690, A topical antifungal agent in development for the treatment of onychomycosis represents a new class of inhibitor and has a novel mechanism of action", Poster No. 769, Annual Meeting of the Society for Investigative Dermatology, Philadelphia, USA, March 3-6, 2006).

The invention thus concerns novel pharmaceutical compositions comprising a **squalene epoxidase inhibitor** in combination or association with a **leucyl-tRNA synthetase inhibitor,** hereinafter briefly named **"the compositions of the invention".**

A suitable **squalene epoxidase inhibitor** is for example an aryl- or heteroarylmethylamine antifungal, preferably of the allyl- or benzylamine class of antifungals, e.g. as described in GB 1'579'879, EP 896, EP 24587, GB 2'116'171, GB 2'185'980, EP 164697, EP 221781 and EP 421302. It is in particular naftifine (Exoderil$^R$) or butenafine (Mentax$^R$), preferably terbinafine (Lamisil$^R$), i.e. (E)-N-methyl-N-(1-naphthylmethyl)-6,6-dimethylhept-2-en-4- amin of formula I

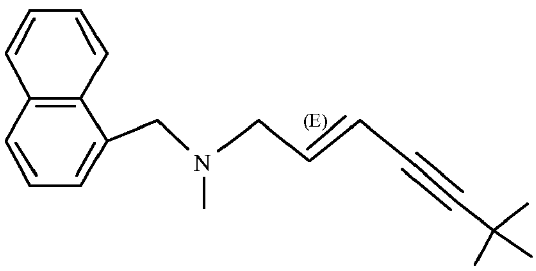

I

in free form or salt form, particularly hydrochloride acid addition salt form, disclosed as Example 16 in EP 24587, or malate acid addition salt form, e.g. the L-(-)-hydrogen malate salt, disclosed as Examples 1 to 3 in e.g. WO 02/070455.

A suitable **leucyl-tRNA synthetase inhibitor** preferably is targetting the editing domain of leucyl-tRNA synthetase, and non-competitively inhibiting that enzyme with respect to ATP and leucine. It is in particular a boron-containing small molecule, such as a disubstituted

2,1-benzoxaborole antifungal, preferably substitued in the 1 and the 5 positions of the benzoxaborole moiety, especially, substituted in the 1 position by hydroxy and in the 5 position by a small moiety such as halogen, methyl, methoxy or cyano.

It preferably is a compound of formula II

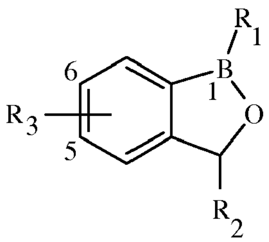

II

wherein

- $R_1$ is hydroxy, phenyl, vinyl or thiophen-3-yl;
- $R_2$ is hydrogen or alkyl of 1 to 4 carbon atoms; and
- $R_3$ is hydrogen, halogen of atomic number from 9 to 35, alkyl of 1 to 4 carbon atoms, alkoxy of 1 to 4 carbon atoms, or cyano;

in free form or in salt form where such forms exist.

Halogen of atomic number from 9 to 35 preferably is fluorine. Alkyl of 1 to 4 carbon atoms preferably is methyl.

Alkoxy of 1 to 4 carbon atoms preferably is methoxy.

$R_1$ preferably is hydroxy.

$R_2$ preferably is hydrogen.

$R_3$ preferably is halogen as defined above or cyano, it especially is fluorine or cyano, more especially fluorine. It preferably is in the 5 position of the 2,1-benzoxaborole moiety.

A preferred subgroup of compounds of formula II is the compounds of formula IIa

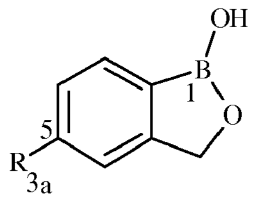

IIa

wherein

$R_{3a}$ is halogen of atomic number from 9 to 35, alkyl of 1 to 4 carbon atoms, alkoxy of 1 to 4 carbon atoms, or cyano,

in free form or in salt form where such forms exist.

Especially preferred is the compound of formula II wherein $R_1$ is hydroxy; $R_2$ is hydrogen; and $R_3$ is in the 5 position and is fluorine, i.e. (5-fluoro-1,3-dihydro-1-hydroxy-2,1-benzoxaborole), hereinafter briefly named **"AN2690".**

The active agents of the compositions of the invention are known or may be obtained according to known processes or to processes analogous to known processes, e.g., as regards 2,1-benzoxaboroles, as described in J.J. Plattner et al., "Medicinal chemistry of AN2690, A novel broad-spectrum antifungal agent in development for the topical treatment of onychomycosis", Poster No. 775, Annual Meeting of the Society for Investigative Dermatology, Philadelphia, USA, March 3-6, 2006).

Particularly preferred are compositions of the invention comprising an arylmethylamine antifungal in combination or association with a 2,1-benzoxaborole antifungal, especially **terbinafine** in combination or association with **AN2690**.

Preferred for use in the treatment of conditions where inflammation is involved, such as atopic dermatitis, acne vulgaris, seborrhoeic dermatitis, rosacea and psoriasis, are compositions of the invention wherein one or both components possess some degree of inherent and-inflammatory activity, such as naftifine or terbinafine in combination with AN2690.

"Treatment" as used herein includes prevention, namely prophylactic as well as curative treatment.

Synergy is e.g. calculated as described in Berenbaum, Clin. Exp. Immunol. 28 (1977) 1, using an interaction term to correct for differences in mechanism between the two drugs, as described in Chou et al., Transpl. Proc. 26 (1994) 3043. The index of synergy is calculated as:

$$\frac{\text{dose of A}}{A_E} + \frac{\text{dose of B}}{B_E} + \frac{(\text{dose of A}) \text{ x } (\text{dose of B})}{A_E \text{ x } B_E}$$

in which the doses of the compounds A and B represent those used in a particular combination, and $A_E$ and $B_E$ are the individual doses of A and B respectively giving the same effect. If the result is less than 1, there is synergy; if the result is 1, the effect is additive; if the result is greater than 1, A and B are antagonistic. By plotting an isobologram of dose of A / $A_E$ vs. dose of B / $B_E$, the combination of maximum synergy can be determined. The synergistic ratio expressed in terms of the ratio by weight of the two compositions at synergistic amounts along the isobologram, especially at or near the point of maximum synergy, can then be used to determine formulations containing an optimally synergistic ratio of the two compounds.

The invention also provides products and methods for co-administration of a squalene epoxidase inhibitor, e.g. terbinafine and a leucyl-tRNA synthetase inhibitor, e.g. AN2690, at synergistically effective dosages, e.g.:

- a method of treatment or prevention of diseases involving a fungal or suspected fungal infection, or a method for immunomodulation or immunosuppression in a condition in which fungal or suspected fungal colonization plays a role or in situations of fungal resistance, in a subject suffering from or at risk for such infection or condition, comprising co-administering synergistically effective amounts of a composition of the invention;
- the use of a squalene epoxidase inhibitor in the manufacture of a medicament for co-administration in synergistically

effective amounts with a leucyl-tRNA synthetase inhibitor;

- the use of a leucyl-tRNA synthetase inhibitor in the manufacture of a medicament for co-administration in synergistically effective amounts with a squalene epoxidase inhibitor;
- a kit of parts comprising a squalene epoxidase inhibitor and a leucyl-tRNA synthetase inhibitor in separate unit dosage forms, preferably wherein the unit dosage forms are suitable for administration of the component compounds in synergistically effective amounts, together with instruction for use, optionally with further means for facilitating compliance with the administration of the component compounds, e.g. a label or drawings;
- the use of a squalene epoxidase inhibitor in the manufacture of a pharmaceutical kit which is to be used for facilitating co-administration with a leucyl-tRNA synthetase inhibitor;
- the use of a leucyl-tRNA synthetase inhibitor in the manufacture of a pharmaceutical kit which is to be used for facilitating co-administration with a squalene epoxidase inhibitor;
- a squalene epoxidase inhibitor and a leucyl-tRNA synthetase inhibitor as a combined pharmaceutical preparation for simultaneous, separate or sequential use, preferably in synergistically effective amounts, e.g. for the treatment or prevention of a fungal infection, such as onychomycosis, or for immunomodulation or immunosuppression in a condition in which fungal or suspected fungal colonization plays a role;
- a pharmaceutical composition comprising a squalene epoxidase inhibitor in combination or association with a leucyl-tRNA synthetase inhibitor, e.g. in synergistically effective amounts, together with at least one a pharmaceutically acceptable diluent or carrier, e.g. for use in treatment or prevention of a fungal infection, such as onychomycosis, or for immunomodulation or immunosuppression in a condition in which fungal or suspected fungal colonization plays a role, or in a situation of fungal resistance; and
- a process for the preparation of a composition of the invention comprising mixing a squalene epoxidase inhibitor and a leucyl-tRNA synthetase inhibitor, in combination or association with at least one pharmaceutically acceptable diluent or carrier.

By "synergistically effective amounts" is meant an amount of squalene epoxidase inhibitor and an amount of leucyl-tRNA synthetase inhibitor which are individually below their respective effective dosages for a relevant indication, but which are pharmaceutically active on co-administration, e.g. in a synergistic ratio, for example as calculated above. Furthermore, "synergistically effective amounts" may mean an amount of squalene epoxidase inhibitor and an amount of a leucyl-tRNA synthetase inhibitor which are individually equal to their respective effective dosages for a relevant indication, and which result in a more than additive effect.

The molar amount of squalene epoxidase inhibitor present is from roughly similar to, to significantly more than the amount of leucyl-tRNA synthetase inhibitor, preferably twice as much or more. Synergistic ratios of squalene epoxidase inhibitor to leucyl-tRNA synthetase inhibitor by weight are thus suitably from about 1:10 to about 50:1, preferably from about 1:5 to about 20:1, most preferably from about 1:1 to about 15:1, e.g. about 2:1 or 1:2.

The compositions of the invention can be administered as a free combination, or the drugs can be formulated into a fixed combination, which greatly enhances the convenience for the patient.

Absolute dosages of the compounds will vary depending on a number of factors, e.g. the individual, the route of administration, the desired duration, the rate of release of the active agent and the nature and severity of the condition to be treated. For example, the amount of active agents required and the release rate thereof may be determined on the basis of known in vitro and in vivo techniques, determining how long a particular active agent concentration in the blood plasma remains at an acceptable level for a therapeutic effect.

For example, in prevention and treatment of fungal or suspected fungal infection, an initial dosage of about 2-3 times the maintenance dosage is suitably administered, followed by a daily dosage of about 2-3 times the maintenance dosage for a period of from one to two weeks, and subsequently the dose is gradually tapered down at a rate of about 5 % per week to reach the maintenance dosage. In general, synergistically effective amounts of terbinafine and AN2690 on oral administration for use in prevention and treatment of fungal diseases in larger animals, e.g. man, are amounts of terbinafine of up to about 50 mg/kg/day, e.g. from about 0.25 mg/kg/day to about 50 mg/kg/day, preferably about 2.5 mg/kg/day, in combination or co-administration with amounts of AN2690 of up to about 2 mg/kg/day, e.g. from about 0.01 mg/kg/day to about 2 mg/kg/day, preferably about 0.5 mg/kg/day, in a synergistic ratio, as described. Suitable unit dosage forms for oral co-administration of these compounds thus may contain on the order of from about 10 mg to about 3000 mg, preferably about 50 mg to about 500 mg of terbinafine, and from about 0.5 mg to about 100 mg, preferably about 3 mg to about 30 mg of AN2690. The daily dosage for oral administration is preferably taken in a single dose, but may be spread out over two, three or four dosages per day. For i.v. administration, the effective dosage is lower than that required for oral administration, e.g. about one fifth the oral dosage.

By "co-administration" is meant administration of the components of the compositions of the invention together or at substantially the same time, e.g. within fifteen minutes or less, either in the same vehicle or in separate vehicles, so that upon oral administration, for example, both compounds are present simultaneously in the gastrointestinal tract.

Preferably, the compounds are administered as a fixed combination.

The compositions of the invention include compositions suitable for administration by any conventional route, in particular compositions suitable for administration either enterally, for example, orally, e.g. in the form of solutions for drinking, tablets or capsules, or parenterally, e.g. in the form of injectable solutions or suspensions; or topically, e.g. for the treatment of fungal conditions of the skin, the nail or mucosae, e.g. in the form of a dermal cream, ointment, ear drops, mousse, shampoo, solution, lotion, gel, emulgel, nail lacquer or like preparation, e.g. in a concentration of from about 0.1 % to about 20 % by weight of each component, especially in combination or association with penetration enhancing agents, as well as for application to the eye, e.g. in the form of an ocular cream, gel or eye-drop preparation, for treatment of fungal or suspected fungal conditions of the lungs and airways, e.g. in the form of inhalable compositions, for mucosal application, e.g. in the form of vaginal tablets, and for application in onychomycosis, e.g. in the form of a nail lacquer.

Topical adminstration, and compositions adapted for topical use in e.g. onychomycosis, such as a nail lacquer, are preferred. However, topical and systemic use may be combined, with one component administered topically, e.g. AN2690, in association with the other component, administered systemically, e.g. terbinafine; or vice-versa.

The compositions of the invention are suitably emulsions, microemulsions, emulsion preconcentrates or microemulsion preconcentrates, or solid dispersions, especially water-in-oil microemulsion preconcentrates or oil-in-water microemulsions, comprising the squalene epoxidase inhibitor and the leucyl-tRNA synthetase inhibitor in a synergistic ratio.

The compositions of the invention can be prepared in conventional manner, e.g. by mixing a squalene epoxidase inhibitor and a leucyl-tRNA synthetase inhibitor, in combination or association with at least one pharmaceutically acceptable diluent or carrier.

The active agent components may be in free form or pharmaceutically acceptable salt form as appropriate.

The following Examples illustrate the invention. The compounds are in free, i.e. neutral or base form unless specified otherwise.

**Example 1: Tablet**

A tablet for oral use with granulated terbinafine hydrochloride and AN2690 powder in form of a solid dispersion is prepared in conventional manner, in a 600 mg dosage, and contains the following ingredients:

| Component | Amount (mg) |
|---|---|
| Terbinafine hydrochloride | 281.25 (corresponds to 250 mg free base) |
| AN2690 | 20.0 |
| silicium dioxide colloidal (Aerosil 200) | 1.95 |
| microcrystalline cellulose | 48.30 |
| sodium carboxymethyl starch | 35.10 |
| hydroxypropylmethyl cellulose 3 cps | 81.70 |
| Poloxamer 188 | 10.00 |
| lactose, anhydrous | 67.50 |
| crospovidone | 50.00 |
| magnesium stearate | 4.20 |
| Total | 600.00 |

**Example 2: Cream**

A cream with dissolved terbinafine base is prepared in conventional manner with AN 26907, both in a 1 % w/w concentration, and contains the following ingredients:

| Component | Amount (g) |
|---|---|
| Terbinafine base | 1.00 |
| AN2690 | 10.00 |
| triglycerides, medium chain | 15.00 |
| oleyl alcohol | 10.00 |
| sodium cetylstearyl sulfate | 1.00 |
| cetyl alcohol | 4.00 |

(continued)

| Component | Amount (g) |
|---|---|
| stearyl alcohol | 4.00 |
| glyceryl monostearate | 2.00 |
| benzyl alcohol | 1.00 |
| propylene glycol | 5.00 |
| citric acid | 0.05 |
| sodium hydroxide | 0.02 |
| water | 44.93 |
| Total | 100.00 |

**Example 3: Ointment**

An ointment with terbinafine hydrochloride and AN 2690 in suspended form is prepared in conventional manner in a 1 % w/w concentration, and contains the following ingredients:

| Component | Amount (g) |
|---|---|
| Terbinafine hydrochloride | 1.125 |
| AN2690 | 10.00 |
| mineral oil | 40.00 |
| petrolatum | 38.875 |
| microcrystalline wax | 10.00 |
| Total | 100.00 |

**Example 4: Vaginal tablet**

A tablet for vaginal use with granulated terbinafine hydrochloride and AN 2690 is prepared in conventional manner, in a 1600 mg dosage, and contains the following ingredients:

| Component | Amount (mg) |
|---|---|
| Terbinafine hydrochloride | 281.25 (corresponds to 250 mg free base) |
| AN2690 | 20.0 |
| lactose monohydrate | 1004.75 |
| sodium carboxymethyl starch | 96.00 |
| hydroxypropylmethyl cellulose 3 cps | 54.00 |
| corn starch | 112.0 |
| magnesium stearate | 32.00 |
| Total | 1600.00 |

**Example 5: Nail lacquer**

A lacquer for use in onychomycosis with terbinafine hydrochloride and AN 2690 is prepared in conventional manner, in a 100 mg dosage, and contains the following ingredients:

| Component | Amount (mg) |
|---|---|
| Terbinafine hydrochloride | 10.0 (corresponds to 8.89 mg free base) |
| AN2690 | 7.5 |
| DDAIP[1)] hydrochloride | 0.5 |
| benzyl alcohol | 0.75 |
| polyvinylpyrrolidone | 0.5 |

(continued)

| Component | | Amount (mg) |
|---|---|---|
| ethanol abs. | | 80.75 |
| | Total | 100.0 |
| 1) dodecyl-2-N,N-dimethylaminopropionate | | |

Terbinafine in Examples 1 to 5 may be replaced by a molar equivalent amount of **tolnaftate, tolciclate, naftifine** or **butenafine.**

AN2690 in Examples 1 to 5 may be replaced with a molar equivalent amount of compound of formula II as depicted above and wherein either

- $R_1$ is hydroxy; $R_2$ is hydrogen; and $R_3$ is hydrogen; or is in the 5 position and is chlorine, methyl, cyano or methoxy; or is in the 4, 6, or 7 position and is fluorine; or
- $R_1$ is hydroxy; $R_2$ is methyl; and $R_3$ is in the 5 position and is fluorine; or
- $R_1$ is phenyl, vinyl or thiophen-3-yl; $R_2$ is hydrogen; and $R_3$ is in the 5 position and is fluorine; i.e. with compound **4a, 4c, 4d, 4e, 4f, 4g, 4h, 4i, 4k, 41, 4m** and **4n,** respectively, in Table I of Poster No. 775 [loc.cit.above]).

## Claims

1. A pharmaceutical composition comprising a squalene epoxidase inhibitor in combination or association with a leucyl-tRNA synthetase inhibitor, together with at least one pharmaceutically acceptable diluent or carrier.

2. A composition according to claim 1 comprising terbinafine in free form or salt form, in combination or association with a compound of formula II

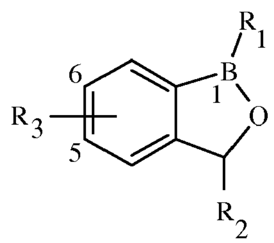

II

wherein

$R_1$ is hydroxy, phenyl, vinyl or thiophen-3-yl;
$R_2$ is hydrogen or alkyl of 1 to 4 carbon atoms; and
$R_3$ is hydrogen, halogen of atomic number from 9 to 35, alkyl of 1 to 4 carbon atoms, alkoxy of 1 to 4 carbon atoms, or cyano;

in free form or in salt form where such forms exist.

3. A composition according to claim 1 comprising terbinafine in free form or salt form, in combination or association with a compound of formula IIa

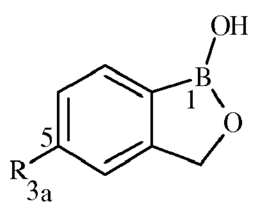

IIa

wherein

$R_{3a}$ is halogen of atomic number from 9 to 35, alkyl of 1 to 4 carbon atoms, alkoxy of 1 to 4 carbon atoms, or cyano,

in free form or in salt form where such forms exist.

**4.** A composition according to any one of claims 1 to 3 comprising terbinafine in free form or salt form, in combination or association with AN2690.

**5.** A method of treatment of a disease involving fungal or suspected fungal infection, or a method for immunomodulation or immunosuppression in a condition in which fungal or suspected fungal colonization plays a role such as onychomycosis, or in a situation of fungal resistance, in a subject suffering from or at risk for such infection or condition, comprising co-administering a synergistically effective amount of a composition according to any one of claims 1 to 3.

**6.** A process for the preparation of a composition according to any one of claims 1 to 3 comprising mixing a squalene epoxidase inhibitor and a leucyl-tRNA synthetase inhibitor, in combination or association with at least one pharmaceutically acceptable diluent or carrier.

**7.** A kit of parts comprising a squalene epoxidase inhibitor and a leucyl-tRNA synthetase inhibitor in separate unit dosage forms, together with instruction for use.

European Patent Office

# PARTIAL EUROPEAN SEARCH REPORT

which under Rule 45 of the European Patent Convention shall be considered, for the purposes of subsequent proceedings, as the European search report

Application Number
EP 06 11 9884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2006/089067 A (ANACOR PHARMACEUTICALS INC [US]; BAKER STEPHEN J [US]; AKAMA TSUTOMU [) 24 August 2006 (2006-08-24) * paragraphs [0002], [0018], [0001] - [0030], [0071] - [0078], [0103], [0110] - [0116], [0135], [0144] - [0147], [0256], [0257]; examples 16-20 * ----- | 1-7 | INV. A61K45/06 A61K31/69 A61K31/137 A61P31/10 |
| Y | BAKER STEPHEN J ET AL: "Discovery of a new boron-containing antifungal agent, 5-fluoro-1,3-dihydro-1-hydroxy-2,1-benzoxaborole (AN2690), for the potential treatment of onychomycosis" JOURNAL OF MEDICINAL CHEMISTRY, vol. 49, no. 15, July 2006 (2006-07), pages 4447-4450, XP002416532 ISSN: 0022-2623 * the whole document * ----- -/-- | 1-7 | |
| | | | TECHNICAL FIELDS SEARCHED (IPC) |
| | | | A61K A61P |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| Munich | 25 January 2007 | Pa l Soto, Raquel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 06 11 9884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/013892 A2 (ANACOR PHARMACEUTICALS INC [US]; LEE VING [US]; PLATTNER JACOB J [US];) 17 February 2005 (2005-02-17)<br>* page 5, line 10 - page 7, line 22 *<br>* page 19, line 16 - page 20, line END *<br>* page 26, line 21 - page 31, line 14 *<br>* examples 11-39 *<br>----- | 1-7 | |
| A | WO 95/33754 A (ZENECA LTD [GB]; AUSTIN PETER WILLIAM [GB]; KNEALE CHRISTOPHER JUAN [G) 14 December 1995 (1995-12-14)<br>* page 1, lines 1-4,35-40 *<br>* page 5, lines 33-37 *<br>* page 6, lines 5-10 *<br>* page 7, lines 27-34 *<br>* page 10, lines 16-19 *<br>* page 11, lines 5-16 *<br>Ex. 64 on pages 22-23* example 1 *<br>----- | 1-7 | TECHNICAL FIELDS SEARCHED (IPC) |
| A | WO 2005/021483 A2 (NOVARTIS AG [CH]; NOVARTIS PHARMA GMBH [AT]; BEUTLER ULRICH [CH]; PENN) 10 March 2005 (2005-03-10)<br>* page 1 *<br>----- | 1-7 | |

3

EPO FORM 1503 03.82 (P04C10)

European Patent Office

**INCOMPLETE SEARCH SHEET C**

Application Number
EP 06 11 9884

Although claim 5 is directed to a method of treatment of the human/animal body (Article 52(4) EPC), the search has been carried out and based on the alleged effects of the composition.

-----

Claim(s) not searched:
5

Reason for the limitation of the search (non-patentable invention(s)):

Article 52 (4) EPC - Method for treatment of the human or animal body by therapy

## ANNEX TO THE EUROPEAN SEARCH REPORT ON EUROPEAN PATENT APPLICATION NO. EP 06 11 9884

This annex lists the patent family members relating to the patent documents cited in the above-mentioned European search report. The members are as contained in the European Patent Office EDP file on
The European Patent Office is in no way liable for these particulars which are merely given for the purpose of information.

25-01-2007

| Patent document cited in search report | Publication date | Patent family member(s) | Publication date |
|---|---|---|---|
| WO 2006089067 A | 24-08-2006 | NONE | |
| WO 2005013892 A2 | 17-02-2005 | AU 2004262524 A1 | 17-02-2005 |
| | | BR PI0411582 A | 08-08-2006 |
| | | CA 2529792 A1 | 17-02-2005 |
| | | CN 1829521 A | 06-09-2006 |
| | | EP 1638578 A2 | 29-03-2006 |
| | | MX PA05013848 A | 04-09-2006 |
| WO 9533754 A | 14-12-1995 | AT 195316 T | 15-08-2000 |
| | | AU 681291 B2 | 21-08-1997 |
| | | AU 2534895 A | 04-01-1996 |
| | | BR 9507966 A | 02-09-1997 |
| | | CA 2190155 A1 | 14-12-1995 |
| | | CN 1155286 A | 23-07-1997 |
| | | DE 69518323 D1 | 14-09-2000 |
| | | DE 69518323 T2 | 01-02-2001 |
| | | EP 0765331 A1 | 02-04-1997 |
| | | JP 10500983 T | 27-01-1998 |
| | | NO 965235 A | 21-01-1997 |
| | | US 5880188 A | 09-03-1999 |
| WO 2005021483 A2 | 10-03-2005 | AU 2004268051 A1 | 10-03-2005 |
| | | BR PI0413896 A | 24-10-2006 |
| | | CA 2536498 A1 | 10-03-2005 |
| | | CN 1845895 A | 11-10-2006 |
| | | EP 1694631 A2 | 30-08-2006 |
| | | IS 8369 A | 23-03-2006 |
| | | MX PA06002348 A | 22-05-2006 |
| | | US 2006076227 A1 | 13-04-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1579879 A
- EP 896 A
- EP 24587 A
- GB 2116171 A
- GB 2185980 A
- EP 164697 A
- EP 221781 A
- EP 421302 A
- WO 02070455 A


### Non-patent literature cited in the description


- **W. MAO et al.** AN2690, A topical antifungal agent in development for the treatment of onychomycosis represents a new class of inhibitor and has a novel mechanism of action. *Poster No. 769, Annual Meeting of the Society for Investigative Dermatology,* 03 March 2006
- **J.J. PLATTNER et al.** Medicinal chemistry of AN2690, A novel broad-spectrum antifungal agent in development for the topical treatment of onychomycosis. *Poster No. 775, Annual Meeting of the Society for Investigative Dermatology,* 03 March 2006
- **BERENBAUM.** *Clin. Exp. Immunol,* 1977, vol. 28, 1
- **CHOU et al.** *Transpl. Proc,* 1994, vol. 26, 3043